# FASCICULE DE BREVET EUROPEEN

(11) **EP 2 785 858 B1**
(45) Date de publication et mention de la délivrance du brevet: **28.10.2015**
(21) Numéro de dépôt: 12806590.1
(22) Date de dépôt: 28.11.2012
(51) Int. Cl.: C12Q 1/37

(54) **DOSAGE DE LA CAPACITÉ DE CONTRÔLE DE C1INH**
VERFAHREN ZUR DOSIERUNG DES KONTROLLFÄHIGKEIT VON C1INH
METHOD FOR DOSING THE CONTROL CAPACITY OF C1INH

(30) Priorité: 28.11.2011 FR 1160851
(43) Date de publication de la demande: 08.10.2014
(73) Titulaire: Universite Joseph Fourier (Grenoble 1), 38041 Grenoble (FR); Centre Hospitalier Universitaire de Grenoble, 38043 Grenoble Cedex 9 (FR)
(72) Inventeur: DROUET, Christian, F-38240 Meylan (FR); GHANNAM, Arije, F-38000 Grenoble (FR); DEFENDI, Federica, F-38240 Meylan (FR); FAVIER, Bertrand, F-38660 St Vincent de Mercuze (FR)
(74) Mandataire: Agasse, Stéphane
(86) Numéro de dépôt international: PCT/FR2012/052743
(87) Numéro de publication internationale: WO 2013/079868

(56) Documents cités:
- EP-A2- 0 224 254
- SCHAPIRA M ET AL: "New and rapid functional assay for C1 inhibitor in human plasma.", BLOOD APR 1982 LNKD- PUBMED:6174161, vol. 59, no. 4, avril 1982 (1982-04), pages 719-724, XP002683264, ISSN: 0006-4971
- DATABASE WPI Week 200316 Thomson Scientific, London, GB; AN 2003-165135 XP002683265, -& RU 2 195 662 C1 (MOSC EPIDEMIOLOGY & MICROBIOLOGY INST) 27 décembre 2002 (2002-12-27)
- WAGENAAR-BOS I G A ET AL: "Functional C1-Inhibitor diagnostics in hereditary angioedema: Assay evaluation and recommendations", JOURNAL OF IMMUNOLOGICAL METHODS, ELSEVIER SCIENCE PUBLISHERS B.V.,AMSTERDAM, NL, vol. 338, no. 1-2, 30 septembre 2008 (2008-09-30), pages 14-20, XP025400183, ISSN: 0022-1759, DOI: 10.1016/J.JIM.2008.06.004 [extrait le 2008-07-23]
- DROUET C ET AL: "A sensitive method to assay blood complement C1 Inhibitor activity", CLINICA CHIMICA ACTA, ELSEVIER BV, AMSTERDAM, NL, vol. 174, no. 2, 31 mai 1988 (1988-05-31), pages 121-130, XP023397341, ISSN: 0009-8981, DOI: 10.1016/0009-8981(88)90379-8 [extrait le 1988-05-31]

## Description

La présente invention concerne une méthode de dosage de la capacité de contrôle de la serpine (SERineProteaseINHhibitor) C1 inhibiteur (C1Inh) plasmatique à partir d'un échantillon de sang d'un patient. L'invention concerne également un kit spécialement conçu pour ce dosage.

La coupure du kininogène de haut poids moléculaire (HK) par la kallicréine (KK) plasmatique (également appelée kininoformation) conduit à la libération de bradykinine (BK). La bradykinine exerce ensuite ses effets par la stimulation du récepteur B2 des kinines exprimé sur les cellules endothéliales. La serpine (SERineProteaseINhibitor) C1 inhibiteur (C1Inh) est un inhibiteur plasmatique de protéase à sérine qui est impliqué dans le contrôle de la formation de BK. Un défaut dans le contrôle exercé par la protéine C1Inh entraîne une surproduction de BK et l'angioedème (AO), s'exprimant par une fuite capillaire avec gonflement des tissus sous-cutanés ou sous-muqueux. La perméabilité vasculaire entraîne chez le patient en situation de crise des oedèmes au niveau de la face et des membres supérieurs, des crampes abdominales accompagnées parfois de vomissements et de diarrhées. La crise provoque également une gêne respiratoire qui peut aller jusqu'à l'asphyxie en cas de localisation laryngée. Dans certains cas sévères d'oedème laryngé, l'angioedème peut conduire à la mort du patient.

Le dosage de la capacité de contrôle de C1Inh permet de diagnostiquer l'angioedème lié au déficit de l'inhibition par C1Inh. Ce dosage permet ensuite de déterminer la quantité de C1Inh à administrer au patient pour rétablir le contrôle de la formation de BK par C1Inh. Il permet également le suivi au cours du temps de la réponse du patient au traitement administré.

Aujourd'hui, la capacité de contrôle de la protéine C1Inh est mesurée dans le plasma des patients par sa capacité à inhiber l'activité estérase de la protéine C1 s. Ce dosage est décrit dans l'article de Drouet *et al*, 1988. L'activité C1s est déterminée par mesure de l'éthanol généré par le clivage d'un substrat synthétique de C1s, le benzoyl-L-Arginine éthyl ester (BAEe). Néanmoins, C1 s n'est pas une protéase impliquée dans la production des kinines, elleest sans impact dans le processus pathologique d'angioedème. En outre, cette méthode de dosage présente l'inconvénient majeur en terme expérimental de nécessiter le prélèvement d'aliquotes au cours de l'incubation, ce qui empêche l'utilisation de la méthode en analyse à haut débit ou en lecture automatisée.

Le principal contrôle de l'activation et de l'activité de la KK est assuré par deux protéines plasmatiques inhibitrices, à savoir les protéines C1inh et α2-Macroglobuline (α2-M). Ainsi, α2-M présente la même capacité de contrôle sur la KK plasmatique que C1Inh et est donc susceptible d'interférer dans une méthode visant le dosage spécifique de la fonction inhibitrice de C1Inh.

L'article de Schapira et al, 1982, décrit le dosage de C1Inh basé sur la capacité de la kallicréine à hydrolyser un substrat chromogène, ledit dosage nécessitant le traitement de l'échantillon par la méthylamine afin d'éliminer les interférences de l'alpha2-Macroglobuline.

La présente invention propose une méthode de dosage de la capacité de contrôle de la protéine C1Inh dans le plasma, qui répond aux attentes susmentionnées.

La présente invention propose une méthode de dosage de la capacité de contrôle de C1Inh qui est corrélée à la pathologie d'angioedème et qui est plus spécifique que celle décrite dans l'article de Drouet *et al,* 1988. Plus précisément, la méthode proposée est basée sur une mesure de la capacité de la KK d'hydrolyser un substrat chromogène ou fluorogène. La KK étant une protéase directement impliquée dans la libération de bradykinine, la méthode de dosage objet de la présente invention est directement corrélée à la pathologie d'angioedème.

En outre, la méthode objet de la présente invention est réalisée dans des conditions telles à permettre le dosage de la protéine C1Inh active, tout en s'affranchissant de l'activité potentielle de l'α2M également présente dans le plasma.

En outre, la méthode de la présente invention permet de doser la capacité de contrôle de la protéine C1Inh, c'est-à-dire la concentration en C1Inh active, c'est-à-dire susceptible d'exercer son activité d'inhibition de la libération de BK. Ceci constitue un avantage important de la présente méthode de dosage, en particulier pour les patients porteurs d'une mutation du gène codant la protéine C1Inh et qui rend la protéine inactive. En effet, dans ce cas, les tests classiques peuvent conduire à des concentrations en C1Inh élevées, alors que la protéine n'est pas active et ne joue pas son rôle dans le contrôle de l'inhibition.

Enfin, la méthode de dosage de la présente invention se fait en un temps, sans transfert après incubation de l'échantillon avec la protéase-cible. Elle est réalisable sur de faibles volumes de plasma. Elle s'applique donc à l'analyse automatisée.

### Méthode de dosage de la capacité de contrôle de C1Inh

La présente invention concerne donc une méthode de dosage de la capacité de contrôle de la protéine C1 inhibiteur plasmatique (C1Inh) à partir d'un échantillon de plasma d'un patient. La méthode de dosage selon l'invention comprend les six étapes suivantes :
a) on prépare un mélange réactionnel à partir de prékallicréine (pKK), de kininogène de haut poids moléculaire (HK) et de protéase facteur Hageman activée (FXIIa), le mélange réactionnel étant ajusté de manière à présenter un pH supérieur à 7 ;
b) on incube l'échantillon de plasma du patient avec un inhibiteur de protéase à sérine pendant une durée au moins égale à 5 minutes, de manière à obtenir un échantillon de plasma sans activité protéasique spontanée et dans lequel ledit inhibiteur est inactivé ou devient inactif vis-à-vis du mélange réactionnel préparé à l'étape a);
c) on incube l'échantillon de plasma obtenue à l'étape b) avec le mélange réactionnel préparé à l'étape a) pendant une durée inférieure ou égale à 20 minutes ;
d) on ajoute à l'échantillon de plasma obtenue à l'étape c) un substrat chromogène ou fluorogène de la KK susceptible de libérer un chromophore ou un fluorophore après hydrolyse par KK;
e) on détecte l'éventuelle libération du chromophore ou fluorophore obtenue à l'étape d) au cours du temps; et
f) on détermine la capacité de contrôle de C1Inh à partir de la détection réalisée à l'étape e).

La méthode de dosage de la présente invention est basée sur l'hydrolyse d'un substrat chromogène ou fluorogène de la KK, protéine dont la présence dans le plasma est directement dépendante de l'activité de C1Inh. Plus précisément, l'activité inhibitrice de C1Inh peut être dosée dans le plasma des patients par mesure de l'activité amidasique de la KKsur un substrat de choix. Un défaut dans le contrôle de C1Inh entraîne une augmentation de libération de KK dans le plasma. Le plasma contient plus de KKet son substrat chromogène ou fluorogène est alors plus fortement hydrolysé. Cette hydrolyse conduit à la libération d'un chromophore ou d'un fluorophore dont la quantité dans l'échantillon peut être détectée. La quantité de chromophore ou fluorophore est directement dépendante de l'activité de la KKet est proportionnelle à celle-ci. Par contre, la quantité de chromophore ou fluorophore dans l'échantillon est indirectement dépendante de l'activité inhibitrice de C1Inh et inversement proportionnelle à celle-ci. Si l'échantillon contient une concentration de C1Inh active importante, celle-ci joue son rôle dans le contrôle de l'inhibition de libération de KK. Dans ce cas, le plasma contient peu de KK, le substrat de celle-ci est faiblement hydrolysé et la quantité de fluorophore ou chromophore dans l'échantillon est faible.

La méthode de la présente invention nécessite la préparation d'un mélange réactionnel et la mise en contact de ce mélange avec un échantillon de plasma dont on souhaite évaluer la capacité de contrôle par C1Inh. Selon l'invention, le mélange réactionnel contient notamment de la pKK et du kininogène de haut poids moléculaire (HK) qui s'associent pour former le complexe pKK/HK. La présence de FXIIa conduit à convertir la pro-enzyme pKK en enzyme active, la KK avec pour conséquence le clivage de HK et la libération de BK. Dans le plasma, l'activation et l'activité de la KK sont sous le contrôle de C1Inh. C1Inh forme un complexe covalent de stoechiométrie 1:1 avec la KK, entrainant la perte complète de l'activité protéolytique et amidolytique. Ainsi, selon la présente invention, on mesure la capacité de contrôle de C1Inh sur l'activité d'hydrolyse de KK. Pour cela on utilise un composé qui est un substrat de KK et dont l'hydrolyse libère un agent détectable (par exemple chromophore ou fluorophore).

Ainsi, par « capacité de contrôle de C1Inh », on entend activité inhibitrice ou fonction inhibitrice de la protéine C1Inh. Cette activité inhibitrice peut notamment être exprimée en mg/l de plasma du patient. La protéine C1Inh est un inhibiteur plasmatique qui est impliqué dans le contrôle de la formation de BK. Un défaut dans le contrôle de C1Inh entraîne une augmentation de la concentration de BKdans le plasma et potentiellement l'angioedème. La protéine C1Inh est donc directement impliquée dans le processus d'angioedème.

Par « patient », on entend un individu humain, qui peut être sain ou malade. La méthode de dosage est réalisée à partir d'un échantillon de plasma du patient.

Selon une première étape de la méthode de dosage selon la présente invention, on prépare un mélange réactionnel à partir des trois réactifs suivants : pKK,HK et protéase facteur Hageman activée (FXIIa).

Selon l'invention, le rapport molaire (mol/mol) pKK/HK est compris entre 1/10 et 10/1. Selon un aspect de l'invention, le rapport molaire pKK/HK est compris entre 1/2 et 2/1. Selon un autre aspect de la présente invention, le rapport molaire pKK/HK du milieu réactionnel est compris entre 3/4 et 3/2. Par exemple, le rapport molaire pKK/HK est de 1.

Selon un aspect de la présente invention, le rapport molaire (mol/mol) pKK/FXIIa est compris entre 1/5 et 1/25. De préférence le rapport molaire pKK/FXIIa est de 1/5.

Le mélange réactionnel est ajusté de manière à présenter un pH supérieur à 7, par exemple compris entre 7 et 8,5. On utilise par exemple des solutions tampons pour ajuster le pH du milieu réactionnel. A titre d'exemple, on peut citer les solutions tampons suivantes : 150 mMNaCl, 50 mMTris-HCl, pH 7.8 ; 150 mMNaCl, 25 mM NaH2PO4, pH 7.6.

Selon un aspect de la présente invention, on prépare un mélange réactionnel qui présente un pH compris entre 7,5 et 8,0, par exemple un pH de 7,8.

Selon un aspect de la présente invention, la première étape de la méthode de dosage consiste à déposer une quantité déterminée (ou un volume déterminé) de chaque réactif du mélange réactionnel dans un récipient adapté, par exemple dans chaque puits d'une plaque de microtitration (e.g. 96-puits ou 384-puits). Ainsi, à la fin de cette étape, et dans la mesure où le récipient utilisé est une plaque de microtitration, chaque puits de la plaque contient un volume identique déterminé de mélange réactionnel. Alternativement, on prépare extemporanément un volume global de mélange réactionnel puis on dépose une quantité déterminée (ou un volume déterminé) de mélange réactionnel dans un récipient adapté, par exemple de façon séquentielle dans une plaque 96-puits.

Selon un aspect de l'invention, le mélange réactionnel est conservé à une température à laquelle les protéines (c'est-à-dire les réactifs du mélange réactionnel) ne présentent pas ou peu d'activité, par exemple à une température inférieure ou égale à 10°C, par exemple à 4°C. Selon cet aspect de l'invention, avant mélange avec le ou les échantillons de plasma du patient en vue du dosage, on incube le mélange réactionnel à une température qui permet l'activation des protéines pendant au moins 5 minutes, par exemple pendant au moins 10 minutes. A titre d'exemple, on place le mélange réactionnel à température ambiante pendant 10 minutes. Cette sous-étape de cet aspect du procédé selon l'invention permet l'activation des protéines du mélange réactionnel. Ainsi selon cet aspect de l'invention, la méthode de dosage comprend notamment :
- une étape qui consiste à préparer un mélange réactionnel à partir de pKK, de HK et de protéase facteur Hageman activée (FXIIa), le mélange réactionnel étant ajusté de manière à présenter un pH supérieur à 7, le mélange étant conservé à une température inférieure à 10°C ; et
- une étape additionnelle qui consiste à placer le mélange réactionnel à température ambiante pendant au moins 5 minutes avant mélange l'ajout de l'échantillon de plasma.

Selon une seconde étape de la méthode de dosage selon la présente invention, on incube l'échantillon de plasma du patient avec un inhibiteur de protéase à sérine pendant une durée au moins égale à 5 minutes, de manière à obtenir un échantillon de plasma sans activité protéasique spontanée et dans lequel ledit inhibiteur est inactivé ou devient inactif vis-à-vis du mélange réactionnel préparé à l'étape a). En effet, l'échantillon de plasma est susceptible de contenir des protéases, notamment de la KK, de la trypsine ou de l'élastase. De par leurs activités amidasiques, ces protéases sont susceptibles d'hydrolyser le substrat chromogène ou fluorogène utilisé dans le cadre de la présente méthode de dosage. Notamment, la présence de KK présente dans le plasma (ou KK plasmatique) est susceptible d'interférer avec le dosage de la KK obtenue à partir de pKK du mélange réactionnel. Il est donc nécessaire d'inhiber ces protéases du plasma, en particulier la KK plasmatique, dans la mesure où leur activité sur le substrat ne peut pas être corrélée à la présence/absence de C1Inh. Pour s'affranchir de cette activité spontanée du plasma vis-à-vis de ce substrat chromogène ou fluorogène qui n'est pas représentative de la capacité de contrôle de C1Inh, on utilise un inhibiteur de protéase à sérine. L'échantillon de plasma du patient est donc incubé avec un inhibiteur de protéase à sérine avant d'être incubé avec le milieu réactionnel.

L'incubation du plasma avec l'inhibiteur de protéases dure au moins 5 minutes. Le temps d'incubation est par exemple d'environ 10 minutes, c'est-à-dire de 10 +/- 1 minutes. Selon un aspect de la présente invention, on incube l'échantillon de plasma avec un inhibiteur de protéases à sérine pendant un temps compris entre 5 et 20 minutes. Selon un autre aspect de la présente invention, on incube l'échantillon de plasma avec un inhibiteur de protéases à sérine pendant au moins 10 minutes.

Selon un aspect de la présente invention, l'inhibiteur de protéase sérique est choisi parmi le *di-iso*propylfluorophosphate (DFP) ou le phenylmethanesulfonylfluoride (PMSF).

Selon un autre aspect encore de la présente invention, la température d'incubation de l'échantillon de plasma avec un inhibiteur de protéase à sérine varie entre 25 et 40°C, par exemple entre 25 et 35°C. La température d'incubation est par exemple d'environ 30°C, c'est-à-dire de 30 +/- 2°C. Une telle température permet l'inactivation de la protéase à sérine pendant un temps d'incubation court.

A l'étape b) de la méthode de la présente invention l'inhibiteur de protéase à sérine est inactivé ou devient inactif lorsqu'il entre au contact du mélange réactionnel de l'étape a). A titre d'exemple, le DFP devient inactif lorsqu'il entre en contact du mélange réactionnel présentant un pH supérieur à 7.

L'opérateur doit s'assurer (1) de l'absence d'activité protéase spontanée (blocage complet par l'inhibiteur irréversible) et que (2) la préparation de DFP ou de PMSF ne laisse pas subsister une quantité excédentaire d'inhibiteur, altérant la mesure d'activité de KK.

Il est à noter que selon la présente invention, l'ordre des étapes a) et b) est indifférent. On peut réaliser d'abord l'étape a) puis l'étape b). Alternativement, on réalise l'étape b) puis l'étape a).

Selon une troisième étape de la méthode de la présente invention, on incube l'échantillon de plasma obtenue à la deuxième étape avec le mélange réactionnel préparé à la première étape pendant une durée inférieure ou égale à 20 minutes.

Ainsi, selon cette étape de la méthode de la présente invention, l'échantillon de plasma qui a été incubé (ou pré-incubé) en présence d'inhibiteur de protéase à sérine, est incubé en présence du mélange réactionnel tel que précédemment préparé.

Ainsi, selon cette étape de la méthode selon l'invention, la protéine C1Inh éventuellement présente dans le plasma du patient est mise au contact du mélange réactionnel contenant les protéines dont les activités sont régulées par C1Inh.

Le temps d'incubation de l'échantillon de plasma avec le milieu réactionnel est inférieur ou égal à 20 minutes, par exemple inférieur ou égal à 15 minutes. Le temps d'incubation est par exemple d'environ 10 minutes, c'est-à-dire de 10 +/- 1 minutes. En effet, les inventeurs se sont rendus compte qu'en incubant le plasma avec le milieu réactionnel pendant une durée inférieure à 20 minutes, il est possible de s'affranchir de la part d'activité de la protéine α2-Macroglobuline (α2M) qui assure le contrôle de l'activation et de l'activité de la KK au même titre que C1Inh. Ceci est dû au fait que l'α2M ne devient active qu'après un certain temps d'incubation en présence des protéines qu'elle régule. Ainsi, en réalisant le dosage après un temps d'incubation inférieur à 20 minutes, les interférences avec l'activité de l'α2M sont négligeables ; le dosage objet de la présente invention correspond à la seule activité de C1Inh. La méthode de dosage selon la présente invention correspond donc uniquement à la capacité de contrôle de la protéine C1Inh. Ceci constitue une caractéristique avantageuse de la présente méthode de dosage.

Selon un autre aspect de la présente invention, la température d'incubation de l'échantillon de plasma avec le milieu réactionnel varie entre 15 et 25°C. La température d'incubation est par exemple celle de la température ambiante.

Selon un aspect de la présente invention, on ajoute un volume déterminé de plasma dans chaque puits d'une plaque de microtitration (e.g. 96-puits ou 384-puits) contenant un volume défini de milieu réactionnel.

Selon un autre aspect encore de la présente invention, le volume de plasma nécessaire pour réaliser le dosage est inférieur ou égal à 2 µl. Par exemple le volume de plasma est de 0,25 µl, 0,5 µl, 0,75 µl, 1 µl, 1,25 µl ou 1,5 µl. Ceci constitue une caractéristique avantageuse de la présente méthode de dosage qui s'applique à de faibles volumes d'échantillons de plasma. Le faible volume de plasma nécessaire pour réaliser le dosage, ainsi que le fait que cet échantillon de plasma soit ajouté a un seul mélange réactionnel permet la mise en oeuvre de la méthode en analyse automatiséé.

Selon un autre aspect de la présente invention, le volume de plasma utilisé est supérieur à 2µl ; par exemple, le volume de plasma est de l'ordre du ml (e.g., 1ml, 10ml, 100ml) ou de l'ordre du litre. Dans ce cas, le rapport volume:Molarité du plasma:prékallicréine est compris entre 1:1 et 4:1 ; par exemple, ce rapport est de 2:1.

Selon une quatrième étape, on ajoute un substrat chromogène ou fluorogène de la KK susceptible de libérer un chromophore ou un fluorophore après hydrolyse par KK substrat chromogène de la KK.

Par « substrat chromogène de la KK», on entend une molécule pouvant être clivée ou modifiée par la KK et qui comprend ou est couplée à un chromophore. Par « chromophore », on entend un groupement d'atomes au sein d'une molécule qui est responsable des propriétés d'absorption et/ou d'émission de lumière dans le domaine de l'ultraviolet, du visible ou de l'infrarouge de cette molécule. Ces propriétés résultent d'une capacité à absorber l'énergie de photons dans une gamme du spectre visible tandis que les autres longueurs d'onde sont transmises ou diffusées. Le substrat chromogène selon l'invention peut être coloré ou incolore. Ce substrat chromogène libère son chromophore sous l'action de la KK.

Par « substrat fluorogène », on entend une molécule pouvant être clivée ou modifiée par la KK et qui comprend ou est couplée à un fluorophore. Ce substrat fluorogène libère son fluorophore sous l'action de KK. Par « fluorophore », on entend un groupement d'atomes au sein d'une molécule qui est responsable de la capacité de cette molécule à émettre de la lumière de fluorescence après excitation. Ce sont généralement des substances composées de plusieurs noyaux aromatiques conjugués ou encore des molécules planes et cycliques qui possèdent une ou plusieurs liaisons n.

Par « substrat fluorogène », on entend également « substrat FRET » (Fluorescence Resonance Energy Transfer ou transfert d'énergie par résonnance de fluorescence), c'est-à-dire une molécule constituée de deux éléments (un fluorophore donneur et un fluorophore accepteur) qui lorsqu'ils sont en résonnance, c'est-à-dire au contact l'un de l'autre avant hydrolyse par la KK, émettent de la fluorescence à une certaine longueur d'onde suite à une excitation. L'action de la KKsépare les deux éléments, ce qui conduit à une perte d'émission de lumière fluorescente à ladite longueur d'onde. L'un des deux éléments constitutif du substrat FRET peut être indépendamment de l'autre élément émettre de la fluorescence à une deuxième longueur d'onde distincte de la fluorescence émise par le substrat FRET. Ainsi le substrat FRET hydrolysé selon l'invention n'émet plus de fluorescence à la première longueur d'onde mais le fluorophore en tant qu'élément indépendamment du substrat FRET émet de la fluorescence à une seconde longueur d'onde.

Les chromophores etfluorophores sont connus de l'homme du métier.

La libération du chromophore ou fluorophore peut être due directement ou indirectement à l'hydrolyse du substrat par la KK. Ainsi, la KKpeut hydrolyser la liaison couplant le substrat au chromophore ou fluorophore, libérant ainsi le chromophore ou fluorophore du substrat. KK peut également hydrolyser un domaine du substrat n'impliquant pas le chromophore ou fluorophore. De préférence, la libération du chromophore ou du fluorophore entraîne un changement de couleur du substrat chromogène ou d'émission de fluorescence du substrat fluorogène. Par conséquent, la détection de la libération du chromophore ou du fluorophore peut en particulier être mise en oeuvre en observant le changement de couleur du substrat chromogène ou d'émission de fluorescence du substrat fluorogène.

De préférence, le substrat chromogène ou fluorogène selon l'invention est un dérivé d'un substrat naturel de la KK.

Selon un aspect de la présente invention, le substrat choisi est un substrat chromogène de la KK et il s'agit du peptide H-D-Pro-Phe-Arg-para-nitroanilide.

Selon une cinquième étape, on détecte l'éventuelle libération du chromophore ou fluorophore obtenue à la quatrième au cours du temps.

Cette étape de la méthode de dosage de la présente invention permet d'obtenir une cinétique de l'activité d'hydrolyse (ou activité amidasique) de la KK au cours du temps. Plus la capacité de contrôle de C1Inh est élevée, plus la cinétique d'hydrolyse est lente et, en particulier, plus la vitesse maximale d'hydrolyse est faible. Au contraire plus la capacité de contrôle de C1Inh est faible, plus la cinétique d'hydrolyse est rapide et, en particulier, plus la vitesse maximale d'hydrolyse est élevée.

La détection (ou mesure) de la libération du chromophore ou du fluorophore peut par exemple être mise en oeuvre en observant le changement de couleur du substrat chromogène ou d'émission de fluorescence du substrat fluorogène.

Selon un aspect de la présente invention, la détection consiste en une lecture de la DO de l'échantillon à la longueur d'onde pertinente.

Dans le cas où le substrat chromogène est le peptide H-D-Pro-Phe-Arg-*para*-nitroanilide, on détecte l'éventuelle libération du chromophore par lecture de la déviation optique (DO) de l'échantillon à une longueur d'onde de 405 nm. En effet, la présence du groupe *para*-nitroanilide ou pNA jouant le rôle de chromophore est détecté par mesure spectrophotométrique à 405nm.

Selon un aspect de la présente invention, on mesure l'éventuelle libération du chromophore ou fluorophore, à partir de l'ajout du substrat chromogène ou fluorogène qui constitue le temps T0 et pendant un temps déterminé, par exemple jusqu'à l'obtention d'une cinétique constante au cours du temps (valeur de cinétique sensiblement constante et correspondant à une phase de plateau sur la courbe de cinétique au cours du temps). A titre d'exemple, on mesure l'éventuelle libération du chromophore ou fluorophore pendant une heure.

Selon une sixième étape, on détermine la capacité de contrôle de C1Inh à partir de la détection réalisée à la cinquième étape.

Cette étape peut être réalisée de manière manuelle ou au moyen d'un programme d'ordinateur, pour la fonction Vmax (%) = ∫(ng C1Inh). Selon un aspect de l'invention, cette étape consiste à reporter la valeur de la vitesse maximale d'hydrolyse déduite de la cinétique d'hydrolyse obtenue à la cinquième étape de la méthode de dosage sur une courbe de titration (courbe de référence). Par « courbe de titration », on entend une courbe de référence obtenue à partir de mesures de vitesse maximale d'hydrolyse effectuées avec des quantités de protéine C1Inh connues, par exemple comprises entre 40 ng et 200 ng de protéine C1Inh.

Selon un autre aspect de l'invention, cette étape consiste à entrer la valeur dans un programme d'ordinateur et à déduire la capacité de contrôle de C1Inh, par exemple au moyen d'un algorithme.

La capacité de contrôle de C1Inh peut par exemple être exprimée en mg/l ou en U/ml (U=20 U/ml), U étant l'unité arbitraire telle que définie dans l'article Drouet *et al.,* 1998..

### Kit ou trousse pour la mise en oeuvre du dosage

La présente invention concerne également un kit ou une trousse pour la mise en oeuvre de la méthode selon l'invention, comprenant :
- de la pKK, du HK et de la protéase facteur Hageman activée (FXIIa),
- des solutions permettant d'obtenir un pH supérieur à 7,
- un inhibiteur de protéase à sérine, et
- un substrat chromogène ou fluorogène de la KK.

La mesure cinétique de la méthode peut être automatisée dans le cadre de son application dans des systèmes d'analyse à haut débit.

### Autres méthodes

La présente invention concerne également une méthode de détermination de la quantité de C1Inh plasmatique nécessaire pour stopper les effets d'un angioedème chez un patient, comprenant :
a) le dosage de la capacité de contrôle de la protéine C1Inh à partir d'un échantillon de plasma du patient, selon l'invention, et
b) la détermination de la quantité de C1Inh à administrer au patient. La présente invention concerne aussi une méthode de détermination de la quantité de stimulateur de C1Inh plasmatique nécessaire pour stopper les effets d'un angioedème chez un patient, comprenant :
   a) le dosage de la capacité de contrôle de la protéine C1Inh à partir d'un échantillon de plasma du patient selon l'invention, et
   b) la détermination de la quantité de stimulateur de C1Inh à administrer au patient.

L'étape b) des deux méthodes de détermination également objet de la présente invention, peut être réalisée par comparaison à des valeurs de référence (patient sain ou même patient en situation non pathologique.

La présente invention concerne également une méthode de suivi de la capacité de contrôle de C1Inh chez un patient au cours du temps consistant à doserà deux temps différents la capacité de contrôle de C1Inh à partir d'un échantillon de plasma de ce patient selon la méthode de dosage de la présente invention.

Cette méthode de suivi peut notamment être mise en place dans les situations suivantes : a) après administration de médicaments destinés à la prophylaxie de l'angioedème (inducteur de la biosynthèse tels que danazol et stanazolol) ; b) après thérapeutique substitutive dans des situations d'angioedème acquis traités par C1Inh purifié ou recombinant ; c) après administration de concentrés de C1Inh appliqués dans des situations inflammatoires (sepsis) ou pour contrer les effets adverses des thérapeutiques antihypertensives (inhibiteurs de l'enzyme de conversion de l'angiotensine-I ; sartans) ; d) après administration de gliptines dans le cadre du traitement du diabète de type II, e) au cours des traitements de l'alopécie ou des suites de chirurgie sur tumeurs de prostate (inhibiteurs de la 5α-réductase).

### Description des figures

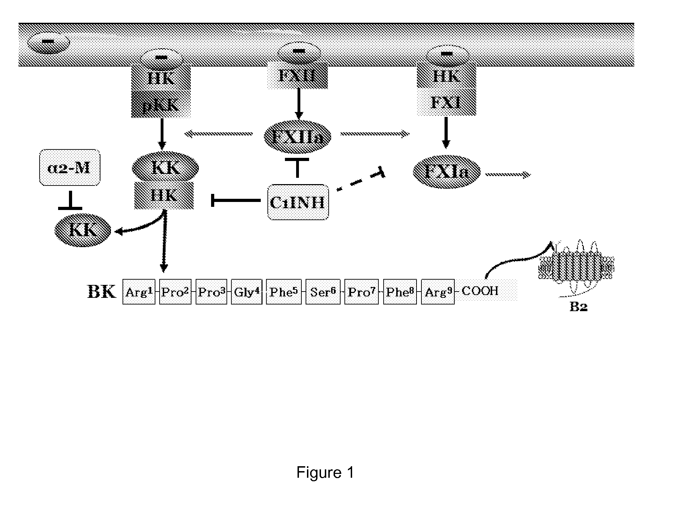

Figure 1 : Formation de la bradykinine endothéliale (kininoformation). C1Inh occupe une position stratégique dans le contrôle de la kininoformation.
Figure 2 : Courbe de titration de la fonction de contrôle de C1Inh. La cinétique de l'activation de la pKK (Vmax) est mesurée en présence de doses croissantes de C1Inh. Incubation de 10 minutes.
   (A) Cinétique de l'activité amidasique en fonction du temps. En abscisse, absorbance (DO à 405 nm) ; en ordonnées, temps en minutes.
   (B) Titration de la fonction de C1Inh par la Vmax de l'activation de la pKK. En abscisses, Vmax en nmol•ml⁻¹•**m**in⁻¹ ; en ordonnées, échantillons FPH avec/sans C1Inh.
      FPH : Produit de l'incubation Facteur XII (F) + Prékallicréine ou PKK (P) + Kininogène de haut poids moléculaire ou HK (H).

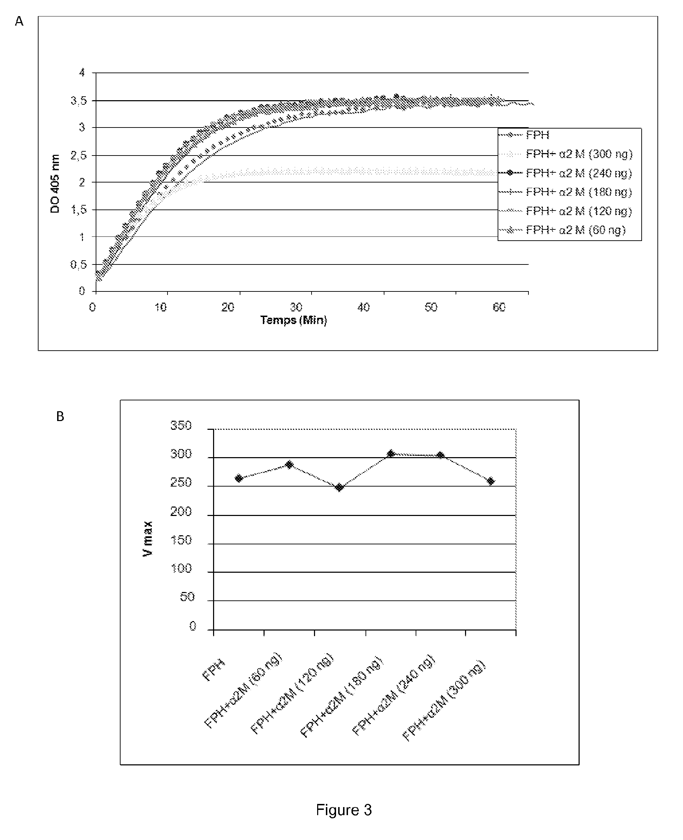

Figure 3 : Courbe de titration de la fonction de l'α2M. La cinétique de l'activation de la pKK (Vmax) est mesurée en présence de doses croissantes d'α2M.
   (A) Cinétique de l'activité amidasique en fonction du temps.En abscisses, absorbance (DO à 405nm) ; en ordonnées, temps en minutes.
   (B) Titration de la fonction de l' α2M par la Vmax de l'activation de la pKK.En abscisse, Vmax en nmol•ml⁻¹•min⁻¹ ; en ordonnées, échantillons FPH avec/sans α2M.

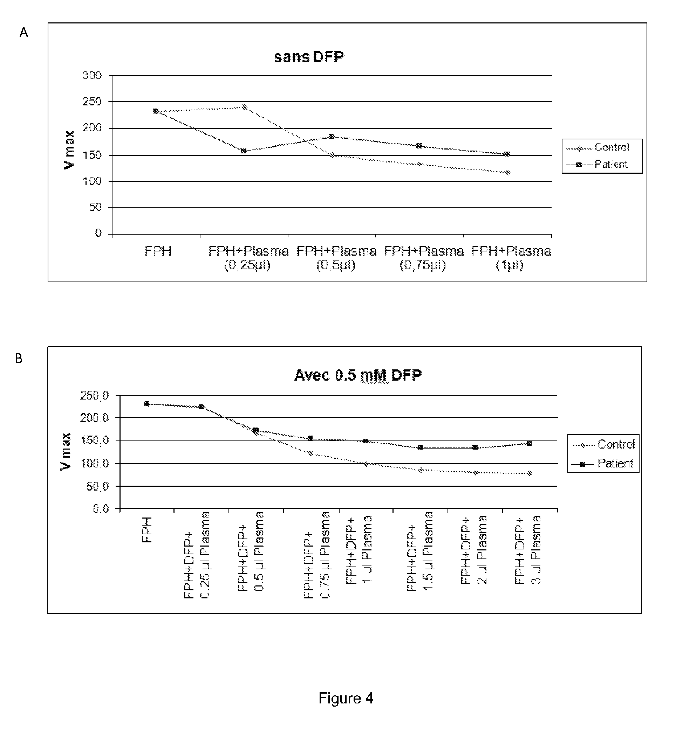

Figure 4 : Vitesse maximale de la cinétique d'activation de la pKK en présence de volumes croissants de plasma non soumis à la pré-incubation par le DFP (A) et soumis à la pré-incubation par le DFP (B).En abscisse, Vmax en nmol•ml⁻¹•min⁻¹ ; en ordonnées, échantillons FPH avec/sans plasma avec/sans DFP.

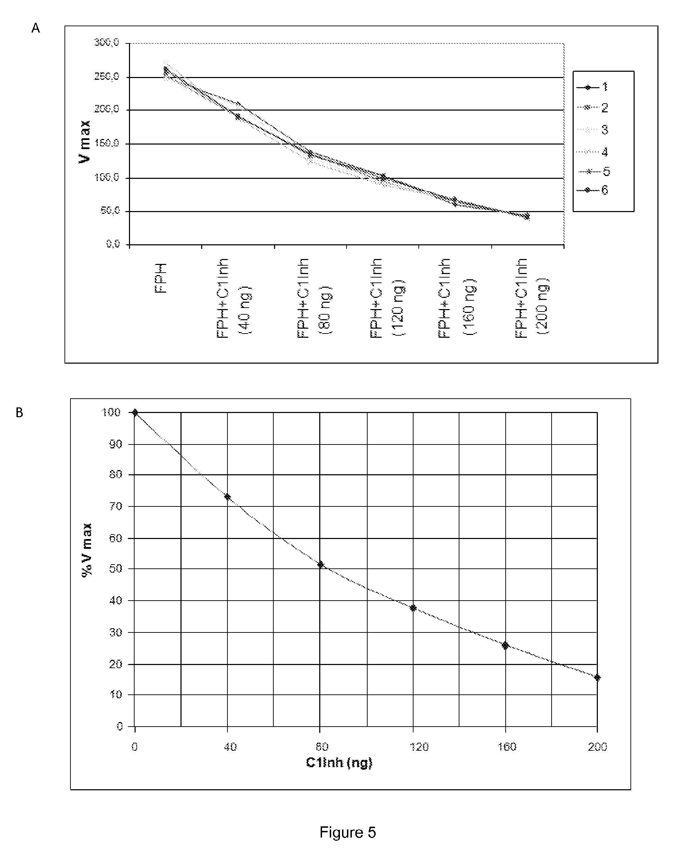

Figure 5 :(A) Reproductibilité du test d'inhibition de l'activation de pKK par des concentrations croissantes de C1Inh. (B)Courbe de titration de C1Inh. Des quantités croissantes de C1Inh sont appliquées dans le test d'activation de la pKK, la Vmax résiduelle Vmax % est calculée par le rapport Vmaxexp/Vmax 100 et la courbe Vmax (%) = ∫(ng C1Inh) est tracée pour la série de mesure de la fonction de C1Inh dans les échantillons.

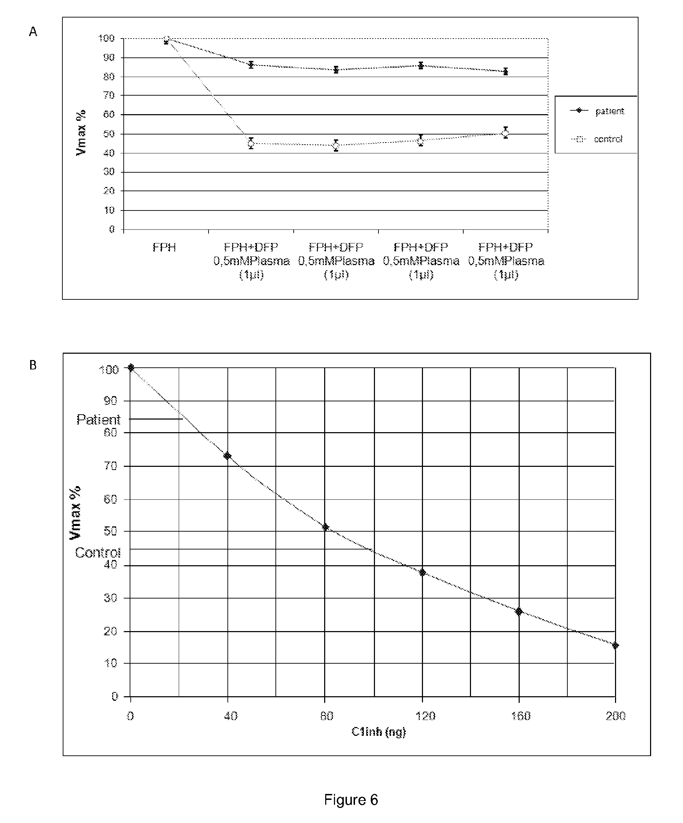

Figure 6 : Mesure de la fonction de C1Inh chez une patiente porteuse de la mutation Arg444Ser. (A) Répétabilité des mesures de la Vmax en présence de l'échantillon (patiente et témoin). (B) Gamme d'étalonnage avec les valeurs de la Vmax résiduelle observée en A.

### Exemples

### Matériels et méthodes

### 1. Echantillonnage

2 x 4,5 mL de sang sont prélevés par ponction veineuse et recueillis dans des tubes contenant du citrate de sodium (0,1 mol/L). Le plasma est recueilli après centrifugation (22°C, 10 min, 2 500g). Le plasma est aliquotéenvolumes de 0,5 ml et congelé à -80°C.

### 2. Protéase et protéines utilisées

La protéase facteur Hageman activée (FXIIa), la prékallicréine (pKK), le kininogène de haut poids moléculaire (HK) proviennent de la société Enzyme ResearchLaboratories Ltd (Swansea UK).
2 pmol de pKK correspondent à 150 ng de protéine (PM 75 000 Da).
2 pmol de HK correspondent à 206,25 ng de protéine (PM 110 000 Da).

La gamme d'étalonnage est établie à partir de C1Inh purifié (BERINERT®, CSL Behring).
2 pmol de C1Inh correspondent à 200 ng de C1Inh purifié (PM 105 000 Da). L'α2M humaine provient de BIOMAC (Leipzig, Allemagne).
0,4 pmol d'α2M correspondent à 300 ng de protéine (PM 720 000 Da).

L'activité de la KKest évaluée par une méthode amidolytique à l'aide d'un substrat chromogène de la KK, le tripeptide H-D-Pro-Phe-Arg-pNA (résidus P3-P1 de la coupure du kininogène, accession ID P01042, positions 387-389). L'hydrolyse du groupe *para*-nitroanilide (pNA) par la KK est détecté par mesure spectrophotométrique à 30°C à 405 nm (ε 405 nm = 8 800 M⁻¹•cm⁻¹). Ce tripeptide provient de la société BACHEM.

### 3. Préparation du mélange réactionnel

Le mélange réactionnel (également appelé FPH) est préparé tel que décrit ci-après : chaque puits de la plaque de microtitration est préalablement saturée par 1% PEG 6000 pendant 1 h à température ambiante. Dans chaque puits d'une plaque de microtitration(plaque 96-puits) à 4°C, sont déposés successivement 150 ng de pKK et 206,25 ng de HK (rapport mol/mol de pKK/HK = 1), puis 25 ng de la protéase FXIIa. Il est préférable de conserver la plaque de microtitration à 4°C de manière à bloquer l'activité des protéines dans le mélange réactionnel. Le volume final dans chaque puits est de 230 µl, ajusté avec du tampon Tris NaCl (NaCl 150 mM, Tris-HCl 50 mM) de manière à ce que le mélange réactionnel présente un pH de 7.8.

Avant l'ajout de C1Inh ou d'échantillon de plasma, on soumet la plaque à une préincubation de 10 minutes à température ambiante.

### 4. Préparation d'une courbe de titration de la capacité de contrôle de C1 Inh

La gamme de C1Inh est établie en concentration décroissante à partir de la concentration stoechiométrique 1/1 (mol/mol) entre C1Inh et pKK.

Dans chaque puits de la plaque de microtitration (plaque 96-puits) préparée selon le paragraphe 3 ci-dessus, sont déposés 200 ng, 160 ng, 120 ng, 80 ng et 40 ng de C1Inh (estimation en quantité de protéine).Le mélange est ensuite soumis à un temps de pré-incubation de 10 minutes à 30°C.

La réaction est déclenchée par l'ajout du tripeptideH-D-Pro-Phe-Arg-*pNA*(0,83 mM final), et l'absorbance est suivie pendant 60 minutes à 30°C à 405 nm sur l'appareil THERMOFISCHER MULTISKAN GO.

### 5. Impact de l'α2-Macroglobuline sur l'activation de la phase contact

Dans chaque puits de la plaque de microtitration (plaque 96-puits) préparée selon le paragraphe 3 ci-dessus, sont déposés des concentrations décroissantes de :
- α2M seule (300 ng, 240 ng, 180 ng, 120 ng et 60 ng ; soit des quantités correspondant à des volumes de 0.05-0.5 µl de plasma humain), et
- un mélange de C1Inh et d'α2M (concentrations décroissantes de chaque serpine).

Le mélange est soumis à une incubation pendant 10 minutes à 37°C.

La réaction est déclenchée par l'ajout du tripeptideH-D-Pro-Phe-Arg-pNA(0,83 mM final), et l'absorbance est suivie pendant 60 minutes à 30°C à 405 nm sur l'appareil THERMOFISCHER MULTISKAN GO.

### 6. Volumes de plasma nécessaire, présence d'un inhibiteur de protéase à sérine

Dans un premier temps, des volumes de plasma d'un patient ou d'un sujet sain (contrôle) variant entre 0,25 et 1 µl sont déposés dans chaque puits de la plaque de microtitration (plaque 96-puits) préparée selon le paragraphe 3 ci-dessus.

Dans un second temps, les dosages sont répétés après incubation avec du *di-iso*propylfluorophosphate (DFP), un inhibiteur de protéase à sérine. En effet, pour s'affranchir de la présence de KK dans le plasma du patient (également appelée activité spontanée du plasma) vis-à-vis du tripeptideH-D-Pro-Phe-Arg-pNA, on soumet les échantillons plasmatiques à unepré-incubation en présence de 0,5 mM de DFP(Sigma), à température ambiante pendant 10 minutes. Le DFP bloque les activités enzymatiques des protéases à sérine, en particulier KK, trypsine et élastase, qui seraient présentes dans le plasma et pourraient interférer avec le dosage (activité spontanée des protéases à sérine, en particulier KK). L'utilisation du DFP est possible dans le cadre de la présente méthode de dosage du fait que cet inhibiteur est hydrolysé lorsqu'il est en contact d'un milieu présentant un pH supérieur à 7 et optionnellement des groupes fonctionnels amines.

Les mélanges sont soumis à l'incubation de 10 min à 37°C.

La réaction est déclenchée par l'ajout du tripeptideH-D-Pro-Phe-Arg-*p*NA(0,83 mM final), et l'absorbance est suivie pendant 60 minutes à 30°C à 405 nm sur l'appareil THERMOFISCHER MULTISKAN GO.

### Résultat et discussion

Courbe de titration C1 Inh, temps d'incubation de C1 Inh dans le mélange réactionnel

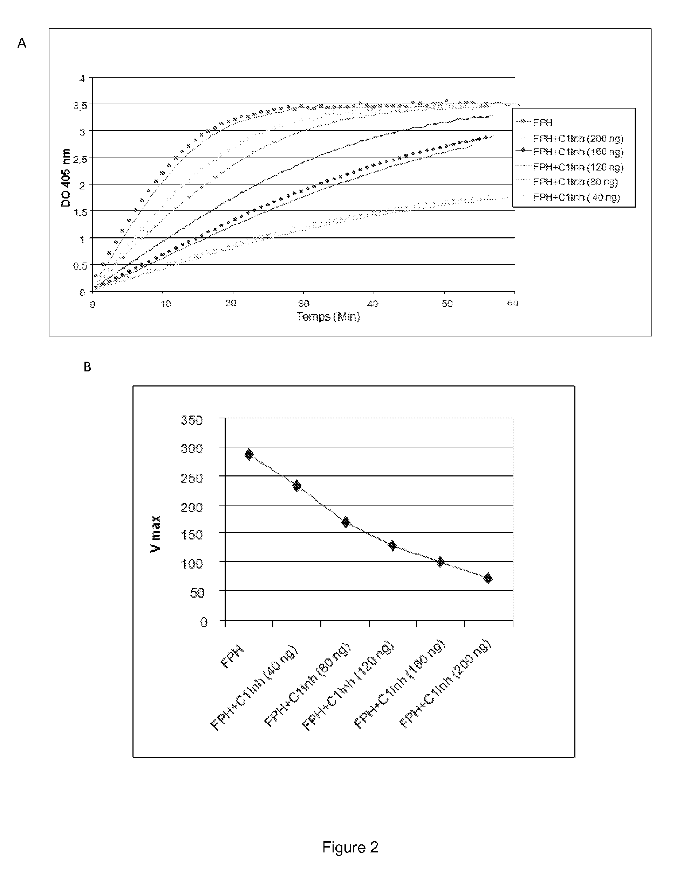

La figure 2A présente les résultats des cinétiques aux différentes concentrations de C1Inh.

La mesure de la Vmax la plus favorable est obtenue pour le temps d'incubation de 10 minutes à 37°C (résultats non montrés). On mesure la vitesse maximale pour chaque concentration.

Tel que montré à la figure 2B, la décroissance de la Vmax est quasi-linéaire avec l'augmentation de la concentration de C1Inh (40-200 ng) : de 233 nmol•ml⁻¹•min⁻¹ (pour 40 ng de C1Inh) à 68 nmol•ml⁻¹•min⁻¹ (pour 200 ng de C1Inh). Le contrôle réalisé sans C1Inh donne une Vmax de 280 nmol•ml⁻¹•min⁻¹.

### Courbe de titration α2-Macroglobuline (α2M) seul ou en présence de C1Inh

Sachant le contrôle possible de l'activation de la pKK par l'α2M, et du fait de la forte concentration de la serpine α2M dans le plasma, son impact sur le système a été testé avec les concentrations 2 fois supérieures à celle de C1Inh pour respecter les conditions du plasma humain.

Les figures 3, en particulier la figure 3B, montrent que, quelle que soit la concentration de l'α2M, la Vmax est de l'ordre de 260 nmol•ml⁻¹•min⁻¹, valeur observée régulièrement comme celle de l'activation de la phase contact dans les conditions retenues. Cette valeur n'est donc pas modifiée par ajout de doses croissantes de l'α2M. L'impact de l'α2M sur l'activation de la phase contact est négligeable pour le temps d'incubation de 10 minutes.

Pour montrer que la fonction de contrôle de l'activation de la pKK par C1Inh, cible du test, n'est pas affectée par la présence de l'autre serpineα2M, le même test que précédemment est exécuté en présence du mélange des deux serpines C1Inh et α2M et en respectant le rapport C1Inh:α2M de 1:2(par mol). La Vmax décroît de façon quasi-linéaire comme pour la figure 2, sans effet ajouté de l'α2M (résultats non montrés). Comparativement aux résultats de la cinétique d'activation de la pKK en l'absence de l'α2M, l'effet de l'α2M n'est pas détectable. Ceci confirme les données de la figure 3.

En conséquence, dans le temps d'incubation de 10 min, l'impact de l'α2M sur le contrôle de l'activation de la pKK est négligeable.

### Evaluation de la quantité optimale de plasma pour la mesure du contrôle par C1Inh

Le même test que précédemment a été exécuté en présence de plasma de sujets sains et de patients pour lesquels la fonction de C1Inh a été reconnue pour être abaissée. Des volumes croissants (0.25-10 µl) ont été appliqués au lieu de l'ajout de l'une ou l'autre des serpines.

L'équivalent C1Inh, utilisé dans le test, du plasma de sujets sains est de 1 µl de plasma représentant 200 à 300 ng de C1Inh. Cette situation correspond au point le plus faible de la Vmax (figure 2B).

La figure 4A montre que l'application du volume de 0,5 µl du plasma patient augmente la Vmax. Cette augmentation est attribuée à la forte activité kininogénase spontanée (74 nmol•ml⁻¹•min⁻¹ ; référence 2.4-10.7 nmol•ml⁻¹•min⁻¹). Tel que montré à la figure 4B, cette activité est inhibée par la pré-incubation du plasma avec 0,5 mM de DFP pendant 10 min à température ambiante. Cette pré-incubation ne modifie pas la Vmax de l'activation de la pKK en présence du plasma. Le DFP ne perturbe pas l'activation de la KK, ni la fonction de contrôle par C1Inh dans les conditions du test (figures 4A et B).

### Reproductibilité des résultats, préparation d'une courbe de titration et application aux échantillons patients

Le test d'inhibition de l'activation de pKK par des concentrations croissantes de C1Inh a été reproduit dans 6 tests indépendants. Voir Figure 5A. Les valeurs obtenues sont toutes groupées autour de la moyenne avec variabilité de 2.5 à 5.5%.

### Données relevées de la Vmax (nmol•ml⁻¹•min⁻¹)

Pour chaque quantité de C1Inh ajoutée: (0) moyenne 256,9, écart-type 7,6 ; (40 ng) moyenne 196,7, écart-type 9,31 ; (80 ng) moyenne 132,6, écart-type 5,45 ; (120 ng) moyenne 97,4, écart-type 5,39 ; (160 ng) moyenne 65,5, écart-type 2,97 ; (200 ng) moyenne 42,2, écart-type 1,08.

L'ajout de concentrations croissantes de C1Inh se traduit par la décroissance quasi-linéaire de la Vmax. Ainsi, ceci permet de considérer l'ajout de C1Inh dans la gamme d'étalonnage du test de l'activation de la pKK en présence de plasma.

Pour ce qui est de la courbe de titration Vmax (%) = ∫(ng C1Inh) (Figure 5B), elle est tracée en utilisant les moyennes des 6 séries de mesure obtenues avec des quantités connues de C1Inh (Figure 6A). La Vmax résiduelle Vmax % est calculée par le rapport Vmaxexp/Vmax 100. Ainsi, par exemple, dans l'échantillon FPH+C1Inh 80 ng, on obtient une valeur de Vmax de 132,6/256,9x100 = 51,6%.

Le faible volume de plasma et l'ajout des composants dans un seul milieu réactionnel autorise la méthode pour des applications en chaîne automatisée. L'application de la méthode de dosage est également possible en développant la procédure avec des volumes plus importants, dès lors que sont respectés les contraintes de concentration des protéines Facteur Xlla,-PKK et HK (FPH)et du rapport 2:1, plasma: pKK(Vol: Mol).

### Dosage comparatif de la capacité de contrôle de C1Inh par le test de l'art antérieur et par celui de la présente invention

### a) Sujet

Le patient est un sujet féminin de 68 ans, avec AOH type Il et porteuse de la mutation Arg444Ser sur C1Inh. Le mutant est considéré comme incapable de contrôler l'activité de la protéase KK.

### b) Concentration en protéine C1Inh

Cette patiente présente une antigénémie(ou concentration) de C1Inh : 503 mg/l (NB : valeurs de référence : 210-345 mg/l). L'antigénémie est mesurée par néphélémétrie (néphélomètreDade Behring BN II).

De cette mesure, on déduit une forte concentration en protéine C1Inh. Compte tenu du fait que la patiente est néanmoins porteuse d'une mutation Arg444Ser sur C1Inh, la question se pose de savoir quel est le pourcentage de C1Inh non mutée, c'est-à-dire en protéine active, dans le sang de la patiente.

### c) Capacité de contrôle de C1Inh dosée au moyen de la technique d'inhibition de l'activité estérasique de la protéase C1 s

L'activité inhibitrice de C1Inh a été mesurée au moyen du test de l'art antérieur décrit dans l'article de Drouet *et al,* 1988(technique d'inhibition de l'activité estérasique de la protéase C1 s). La mesure de la fonction réalisée par la méthode de la présente invention s'avère être possible dans des échelles de valeurs inférieures au seuil de détection, c'est-à-dire <2 U/ml ou <30 mg/L (NB : valeurs de référence 17.2-27.4 U/ml).

### d) Capacité de contrôle de C1Inh dosée au moyen de la méthode de la présente invention

On déduit de la Figure 6A que la Vmax du patient est de 85% et la Vmax du témoin est de 45%. Ces deux valeurs de V-max transposées sur la courbe de titration de la figure 6B permettent d'évaluer l'activité équivalente de C1Inh.

Le patient développe l'activité équivalente C1Inh de 22 ng, pour un témoin expérimental de 100 ng. La fonction de C1Inh plasmatique est donc de 22%. L'activité équivalente C1Inh de 22 ngest celle mesurée dans un volume de 1µl et correspond donc à une concentration de 22mg/l.

Ainsi la mesure réalisée par la méthode de la présente invention conduit à une valeur de capacité de contrôle de C1Inh de 22mg/l. Cette valeur est à comparer à la valeur obtenue selon la technique d'inhibition de l'activité estérasique de la protéase C1 s, <30 mg/L. La technique de dosage de l'art antérieur ne permet pas d'obtenir une valeur précise de la capacité de contrôle de C1Inh dans des échelles aussi faibles. Ainsi le test de l'art antérieur ne permet pas le suivi de l'évolution dans le temps de la capacité de contrôle de C1Inh.

On note en outre que le dosage réalisé par le biais des anticorps anti-C1Inh indique une concentration en C1Inh de 503 mg/l. Ainsi, sur la totalité des molécules C1Inh présente dans le plasma de la patiente, seule une petite partie est active, environ 4%.

Remarque : Pour le volume de 1 µl, il est attendu que la mesure de Vmax s'associe à la valeur d'antigénémie équivalente C1Inh de 200 ng. La Vmax résiduelle expérimentale est de 100 ng soit la moitié de la valeur attendue. Cette valeur expérimentale est de moitié celle attendue par la courbe de titration ; pour expliquer cette observation, est émise l'hypothèse de l'absence de corrélation directe entre la protéine C1Inh purifiée (utilisée dans la courbe de titration) et la protéine C1Inh plasmatique (engageant des liaisons avec des protéines du plasma, avec perte d'une partie de sa réactivité).

## Revendications

1. Méthode de dosage de la capacité de contrôle de la protéine C1 inhibiteur plasmatique (C1Inh) à partir d'un échantillon de plasma d'un patient, comprenant les étapes suivantes :
a) on prépare un mélange réactionnel à partir de prékallicréine (pKK), de kininogène de haut poids moléculaire (HK) et de protéase facteur Hageman activée (FXIIa), le mélange réactionnel étant ajusté de manière à présenter un pH supérieur à 7 ;
b) on incube l'échantillon de plasma du patient avec un inhibiteur de protéase à sérine pendant une durée au moins égale à 5 minutes, de manière à obtenir un échantillon de plasma sans activité protéasique spontanée ; et dans lequel ledit inhibiteur est inactivé ou devient inactif vis-à-vis du mélange réactionnel préparé en a).
c) on incube l'échantillon de plasma obtenue à l'étape b) avec le mélange réactionnel préparé à l'étape a) pendant une durée inférieure ou égale à 20 minutes ;
d) on ajoute à l'échantillon de plasma obtenue à l'étape c) un substrat chromogène ou fluorogène de la kallicréine (KK) susceptible de libérer un chromophore ou un fluorophore après hydrolyse par KK;
e) on détecte l'éventuelle libération du chromophore ou fluorophore obtenue à l'étape d) au cours du temps; et
f) on détermine la capacité de contrôle de C1Inh à partir de la détection réalisée à l'étape e).

2. Méthode de dosage selon la revendication 1, selon laquelle le rapport (mol/mol) pKK/HK du mélange réactionnel est compris entre 1/10 et 10/1, de préférence exemple entre 1/2 et 2/1.

3. Méthode de dosage selon l'une quelconque des revendications précédentes, selon laquelle le mélange réactionnel présente un pH compris entre 7 et 8,5.

4. Méthode de dosage selon l'une quelconque des revendications précédentes, selon laquelle l'inhibiteur de protéase à sérine est choisi parmi le *di-iso*propylfluorophosphate (DFP) ou le phenylmethanesulfonylfluoride (PMSF).

5. Méthode de dosage selon l'une quelconque des revendications précédentes, selon laquelle à l'étape d) on utilise le peptide H-D-Pro-Phe-Arg-*para*-nitroanilide comme substrat chromogène de la KK et à l'étape e) on détecte l'éventuelle présence du chromophore pNA par lecture spectrophotométrique à 405 nm.

6. Méthode de dosage selon l'une quelconque des revendications précédentes, selon laquelle le volume de plasma nécessaire pour réaliser le dosage est inférieur à 2 µl.

7. Kit pour la mise en oeuvre de la méthode selon l'une quelconque des revendications 1 à 6, comprenant :
- de la prékallicréine (pKK), du kininogène de haut poids moléculaire (HK) et de la protéase facteur Hageman activée (FXIIa),
- des solutions permettant d'obtenir un pH supérieur à 7,
- un inhibiteur de protéase à sérine, et
- un substrat chromogène ou fluorogène de la KK.

8. Méthode de détermination de la quantité de C1Inh plasmatique nécessaire pour stopper les effets d'un angioedème chez un patient comprenant :
a) le dosage de la capacité de contrôle de la protéine C1Inh à partir d'un échantillon de plasma du patient, selon l'une quelconque des revendications 1 à 6, et
b) la détermination de la quantité de C1Inh à administrer au patient.

9. Méthode de détermination de la quantité de stimulateur de C1Inh plasmatique nécessaire pour stopper les effets d'un angioedème chez un patient, comprenant :
a) le dosage de la capacité de contrôle de la protéine C1Inh à partir d'un échantillon de plasma du patient selon l'une quelconque des revendications 1 à 6, et
b) la détermination de la quantité de stimulateur de C1Inh à administrer au patient.

10. Méthode de suivi de la capacité de contrôle de C1Inh chez un patient au cours du temps consistant à doser à deux temps différents la capacité de contrôle de C1Inh à partir d'un échantillon de plasma de ce patient selon la méthode de l'une quelconque des revendications 1 à 6.

## Patentansprüche

1. Verfahren zur Dosierung der Kontrollkapazität des Proteins C1-Inhibitor (C1Inh) im Plasma aus einer Plasmaprobe eines Patienten, wobei das Verfahren die folgenden Schritte umfasst:
a) Herstellen eines Reaktionsgemischs aus Präkallikrein (pKK), hochmolekularem Kininogen (HK) und der Protease aktivierter Hageman-Faktor (FXIIa), wobei das Reaktionsgemisch derart eingestellt wird, dass es einen pH-Wert von mehr als 7 aufweist;
b) Inkubieren der Plasmaprobe des Patienten mit einem Serinprotease-Inhibitor für einen Zeitraum von mindestens gleich 5 Minuten, um eine Plasmaprobe ohne spontane Proteaseaktivität zu erhalten; und wobei der besagte Inhibitor gegenüber dem in a) hergestellten Reaktionsgemisch inaktiviert ist oder inaktiv wird;
c) Inkubieren der in Schritt b) erhaltenen Plasmaprobe mit dem in Schritt a) hergestellten Reaktionsgemisch für einen Zeitraum kleiner gleich 20 Minuten;
d) Zugeben eines chromogenen oder fluorogenen Substrats von Kallikrein (KK) zu der in Schritt c) erhaltenen Plasmaprobe, das ein Chromophor oder ein Fluorophor nach Hydrolyse durch KK freisetzen kann;
e) Nachweisen der etwaigen in Schritt d) erhaltenen Freisetzung des Chromophors oder Fluorophors im Zeitablauf; und
f) Bestimmen der Kontrollkapazität von C1Inh aus dem in Schritt e) durchgeführten Nachweis.

2. Dosierungsverfahren nach Anspruch 1, wobei das pKK:HK-Verhältnis (Mol:Mol) des Reaktionsgemischs zwischen 1:10 und 10:1, vorzugsweise beispielsweise zwischen 1:2 und 2:1 liegt.

3. Dosierungsverfahren nach einem der vorhergehenden Ansprüche, wobei das Reaktionsgemisch einen pH-Wert aufweist, der zwischen 7 und 8,5 liegt.

4. Dosierungsverfahren nach einem der vorhergehenden Ansprüche, wobei der Serinprotease-Inhibitor aus Diisopropylfluorphosphat (DFP) oder Phenylmethansulfonylfluorid (PMSF) ausgewählt ist.

5. Dosierungsverfahren nach einem der vorhergehenden Ansprüche, wobei in Schritt d) das Peptid H-D-Pro-Phe-Arg-para-Nitroanilid als chromogenes Substrat von KK verwendet wird und in Schritt e) das mögliche Vorliegen des Chromophors pNA durch spektralphotometrisches Auslesen bei 405 nm nachgewiesen wird.

6. Dosierungsverfahren nach einem der vorhergehenden Ansprüche, wobei das zum Durchführen der Dosierung erforderliche Plasmavolumen kleiner als 2 µl ist.

7. Kit zur Umsetzung des Verfahrens nach einem der Ansprüche 1 bis 6, wobei das Kit Folgendes umfasst:
- Präkallikrein (pKK), hochmolekulares Kininogen (HK) und die Protease aktivierter Hageman-Faktor (FXIIa),
- Lösungen, die das Erhalten eines pH-Werts von mehr als 7 ermöglichen,
- einen Serinprotease-Inhibitor, und
- ein chromogenes oder fluorogenes Substrat von KK.

8. Verfahren zur Bestimmung der Menge von C1Inh im Plasma, die zum Stoppen der Effekte eines Angioödems bei einem Patienten erforderlich ist, wobei das Verfahren Folgendes umfasst:
a) Dosierung der Kontrollkapazität des Proteins C1Inh aus einer Plasmaprobe des Patienten nach einem der Ansprüche 1 bis 6, und
b) Bestimmung der an den Patienten zu verabreichenden C1Inh-Menge.

9. Verfahren zur Bestimmung der Menge von Stimulator von C1Inh im Plasma, die zum Stoppen der Effekte eines Angioödems bei einem Patienten erforderlich ist, wobei das Verfahren Folgendes umfasst:
a) Dosierung der Kontrollkapazität des Proteins C1Inh aus einer Plasmaprobe des Patienten nach einem der Ansprüche 1 bis 6, und
b) Bestimmung der an den Patienten zu verabreichenden C1Inh-Stimulator-Menge.

10. Verfahren zur Beobachtung der Kontrollkapazität von C1Inh bei einem Patienten im Zeitablauf, wobei das Verfahren in dem Dosieren der Kontrollkapazität von C1Inh zu zwei verschiedenen Zeiten aus einer Plasmaprobe dieses Patienten gemäß dem Verfahren nach einem der Ansprüche 1 bis 6 besteht.

## Claims

1. A method for assaying the control capacity of the plasma protein C1 inhibitor (C1Inh) from a plasma sample of a patient, comprising the following steps of:
a) preparing a reaction mixture from prekallikrein (pKK), high molecular weight kininogen (HK) and activated Hageman factor protease (FXIIa), the reaction mixture being adjusted so as to present a pH greater than 7;
b) incubating the plasma sample of the patient with a serine protease inhibitor during a period of at least 5 minutes, so as to obtain a plasma sample without spontaneous protease activity; and wherein said inhibitor is inactivated or becomes inactive regarding the reaction mixture prepared in a).
c) incubating the plasma sample obtained in step b) with the reaction mixture prepared in step a) during a period less than or equal to 20 minutes;
d) adding to the plasma sample obtained in step c) a chromogenic or fluorogenic substrate of kallikrein (KK) likely to release a chromophore or a fluorophore after hydrolysis by KK;
e) detecting the possible release of the chromophore or fluorophore obtained in step d) over time; and
f) determining the capacity to control C1Inh from the detection carried out in step e).

2. The assay method according to claim 1, according to which the ratio (mol/mol) pKK/HK of the reaction mixture is comprised between 1/10 and 10/1, preferably for example between 1/2 and 2/1.

3. The assay method according to any one of the preceding claims, according to which the reaction mixture has a pH comprised between 7 and 8.5.

4. The assay method according to any one of the preceding claims, according to which the serine protease inhibitor is selected from the di-isopropylfluorophosphate (DFP) or the phenylmethanesulfonylfluoride (PMSF).

5. The assay method according to any one of the preceding claims, according to which in step d) the peptide H-D-Pro-Phe-Arg-para-nitroanilide is used as a chromogenic substrate of KK and in step e) the possible presence of pNA chromophore is detected by spectrophotometric reading at 405 nm.

6. The assay method according to any one of the preceding claims, according to which the volume of plasma required to perform the assay is less than 2µl.

7. A Kit for implementing the method according to any one of claims 1 to 6, comprising:
- prekallikrein (pKK), high molecular weight kininogen (HK) and activated Hageman factor protease (FXIIa),
- solutions allowing obtaining a pH greater than 7,
- serine protease inhibitor, and
- chromogenic or flurogenic substrate of KK.

8. A method for determining the required amount of plasma C1Inh to stop the effects of an angioedema in a patient, comprising:
a) assaying the capacity to control C1Inh protein from a plasma sample of the patient, according to any one of claims 1 to 6, and
b) determining the amount of C1Inh to be administrated to the patient.

9. The method for determining the required amount of plasma C1Inh stimulator to stop the effects of an angioedema in a patient, comprising:
a) assaying the capacity to control C1Inh protein from a plasma sample of the patient according to any one of claims 1 to 6, and
b) determining the amount of C1Inh stimulator to be administrated to the patient.

10. A method for monitoring the control capacity of C1Inh in a patient over time consisting of assaying, in two different times, the capacity to control C1Inh from a plasma sample of this patient according to the method of any one of claims 1 to 6.
